# EUROPÄISCHE PATENTANMELDUNG

(11) **EP 0 564 960 A1**
(43) Veröffentlichungstag der Anmeldung: **13.10.1993**
(21) Anmeldenummer: 93105254.2
(22) Anmeldetag: 30.03.1993
(51) Int. Cl.: C07D 471/04, A61K 31/435

(54) **Imidazopyridine als Angiotensin II antagonisten**

(30) Priorität: 06.04.1992 DE 4211474
(71) Anmelder: MERCK PATENT GmbH, D-64271 Darmstadt (DE)
(72) Erfinder: Mederski, Werner, Dr., W-6105 Erzhausen (DE); Dorsch, Dieter, Dr., W-6105 Ober-Ramstadt (DE); Beier, Norbert, Dr., W-6107 Reinheim 5 (DE); Schelling, Pierre, Prof. Dr., W-6109 Mühltal (DE); Lues, Ingeborg, Dr., W-6100 Darmstadt (DE); Minck, Klaus-Otto, Dr., W-6105 Ober-Ramstadt (DE)

(57) **Zusammenfassung**

Imidazopyridinderivate der Formel I 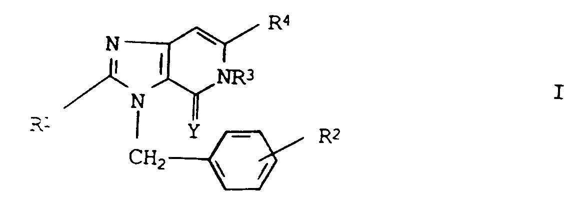
worin
R¹ bis R⁴ und Y die in Patentanspruch 1 angegebene Bedeutung haben,
sowie deren Salze zeigen Angiotensin II-antagonistische Eigenschaften und wirken u.a. blutdrucksenkend.

## Beschreibung

Die Erfindung betrifft neue Imidazopyridinderivate der Formel I 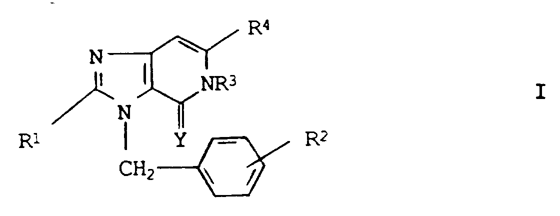
worin
- R¹: H oder A,
- R²: H, Hal, OH, OA, COOH, COOA, CONH₂, CN, NO₂, NH₂, NHA, N(A)₂, NHCOR⁵, NHSO₂R⁵ oder 1H-5-Tetrazolyl,
- R³: H, Cyan-alkyl, Ar-alkyl, Cycloalkyl-alkyl mit 3-8 C-Atomen in der Cycloalkylgruppe, Het-alkyl, Ar'-alkyl, R⁶-CO-alkyl, Ar-CO-alkyl oder Het-CO-alkyl mit jeweils 1-6 C-Atomen im "alkyl"-Teil, wobei ein H-Atom im "alkyl"-Teil durch eine COOH- oder eine COOA-Gruppe substituiert sein kann,
- R⁴: H oder Hal,
- R⁵ und R⁶: jeweils Alkyl mit 1-6 C-Atomen, worin auch ein oder mehrere H-Atom(e) durch F ersetzt sein kann (können),
- Y: O oder S,
- A: Alkyl, Alkenyl oder Alkinyl mit jeweils bis zu 6 C-Atomen,
- Ar: eine unsubstituierte oder eine durch Hal, R⁵, OH, OA, COOH, COOA, CN, NO₂, NH₂, NHA, N(A)₂, NHCOR⁵, NHSO₂R⁵ oder 1H-5-Tetrazolyl mono-substituierte Phenylgruppe,
- Ar': eine durch Ar substituierte Phenylgruppe,
- Het: einen fünf- oder sechsgliedrigen heteroaromatischen Rest mit 1 bis 3 N-, O- und/oder S-Atomen, der auch mit einem Benzol- oder Pyridinring kondensiert sein kann und
- Hal: F, Cl, Br oder I bedeuten,
sowie ihre Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem zur Behandlung der angiotensin II-abhängigen Hypertension, des Aldosteronismus und der Herzinsuffizienz sowie von Störungen des Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie. Diese Wirkungen können nach üblichen in vitro- oder in vivo-Methoden ermittelt werden, wie sie z.B. in der US-PS 4 880 804 und in der WO 91/14367 beschrieben sind, ferner von A.T. Chiu et al., J Pharmacol. Exp. Therap. 250, 867-874 (1989), und von P.C Wong et al., ibid. 252, 719-725 (1990; in vivo, an Ratten).

Gegenstand der Erfindung sind die Verbindungen der Formel I, ihre Salze sowie ein Verfahren zur Herstellung dieser Verbindungen und ihrer Salze, dadurch gekennzeichnet, daß man eine Verbindung der Formel II 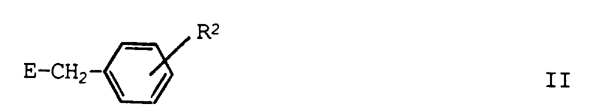
worin
- E: Cl, Br, I oder eine freie oder reaktionsfähig funktionell abgewandelte OH-Gruppe bedeutet und
- R²: die in Anspruch 1 angegebene Bedeutung hat,
mit einer Verbindung der Formel III 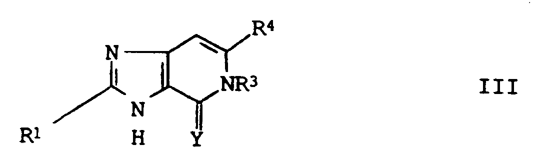
worin
R¹, R³, R⁴ und Y die in Anspruch 1 angegebenen Bedeutungen haben,
umsetzt
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹, R², R³, R⁴ und/oder Y in einen oder mehrere andere Reste R¹, R², R³, R⁴ und/oder Y umwandelt und/oder eine Base oder Säure der Formel I in eines ihrer Salze umwandelt.

Vor- und nachstehend haben die Reste bzw. Parameter R¹ bis R⁸, Y, A, Ar, Ar', Het, Hal und E die bei den Formeln I und II angegebenen Bedeutungen, falls nicht ausdrücklich etwas anderes angegeben ist.

In den vorstehenden Formeln bedeutet A insbesondere Alkyl mit 1-6, vorzugsweise 1, 2, 3, oder 4 C-Atomen, vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethyl-propyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. A kann aber auch Alkenyl oder Alkinyl mit jeweils 2-6, vorzugsweise 2, 3 oder 4 C-Atomen bedeuten, insbesondere Vinyl, 1- oder 2-Propenyl (Allyl), 1-Propen-2-yl, 1-, 2- oder 3-Butenyl, Ethinyl, 1- oder 2-Propinyl (Propargyl), 1-, 2- oder 3-Butinyl.

Dementsprechend ist der Rest OA vorzugsweise Methoxy, weiterhin Ethoxy, Propoxy, Isopropoxy, Butoxy, Isobutoxy, sek.-Butoxy, tert.-Butoxy, Vinyloxy, Allyloxy, Ethinyloxy oder Propargyloxy. Die Gruppe COOA ist bevorzugt Methoxycarbonyl oder Ethoxycarbonyl, ferner Propyloxycarbonyl, Isopropyloxycarbonyl, Butyloxycarbonyl, Isobutyloxycarbonyl, Allyloxycarbonyl, Propargyloxycarbonyl. Die Gruppe NHA ist bevorzugt Methylamino oder Ethylamino. Die Gruppe N(A)₂ ist bevorzugt Dimethylamino oder Diethylamino.

Hal bedeutet vorzugsweise F, Cl oder Br, aber auch J.

Der Rest Ar ist vorzugsweise eine unsubstituierte Phenylgruppe, weiterhin bevorzugt eine in p-Stellung substituierte, aber auch in o- oder m-Stellung substituierte Phenylgruppe. Bevorzugte Substituenten sind COOH, COOA, NO₂ und 1H-5-Tetrazolyl. Dementsprechend ist Ar bevorzugt Phenyl, o-, m- oder (insbesondere) p-Carboxyphenyl, o-, m- oder (insbesondere) p-Methoxycarbonylphenyl, o-, m- oder (insbesondere) p-Ethoxycarbonylphenyl, o-, m- oder (insbesondere) p-Nitrophenyl, o-, m- oder (insbesondere) p-(1H-5-Tetrazolyl)-phenyl, ferner bevorzugt o-, m- oder (insbesondere) p-Aminophenyl, o-, m- oder (insbesondere) p-Dimethylaminophenyl, o-, m- oder (insbesodere) p-Diethylaminophenyl, o-, m- oder p-Tolyl, o-, m- oder p-Trifluormethylphenyl, o-, m- oder p-Hydroxyphenyl, o-, m- oder p-Methoxyphenyl, o-, m- oder p-Fluorphenyl, o-, m- oder p-Chlorphenyl, o-, m- oder p-Bromphenyl, o-, m- oder p-Iodphenyl, o-, m- oder p-Cyanphenyl, o-, m- oder p-Methylaminophenyl, o-, m- oder p-Acetamidophenyl, o-, m- oder p-Trifluoracetamidophenyl, o-, m- oder p-Methylsulfonamidophenyl, o-, m- oder p-Trifluormethylsulfonamidophenyl.

Der Rest Ar' ist bevorzugt 4-Biphenylyl, 2'-Carboxy-4-biphenylyl, 2'-Methoxycarbonyl-4-biphenyly, 2'-Cyan-4-biphenylyl oder 2'-(1H-5-Tetrazolyl)-4-biphenylyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

In den Begriff "Het" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z. B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl, 4-Methyl-5-pyrazolyl, 4- oder 5-Methyl-3-isoxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Der Rest Y ist vorzugsweise O.

Der Rest R¹ ist vorzugsweise A, insbesondere Butyl, ferner bevorzugt Propyl, Pentyl oder Hexyl.

Der Rest R² ist vorzugsweise COOH, ferner bevorzugt 1H-5-Tetrazolyl, COOCH₃, COOC₂H₅, CONH₂, CN oder NO₂.

Im Rest R³ steht der "alkyl"-Teil in den einzelnen Gruppen vorzugsweise für -CH₂- oder -CH₂CH₂-, ferner bevorzugt für -CH(CH₃)-, -(CH₂)₃-, -(CH₂)₄-, -(CH₂)₅- oder -(CH₂)₆-. Im einzelnen steht R³ bevorzugt für H; Ar-alkyl wie Benzyl, 1- oder 2-Phenylethyl, o-, m- oder (insbesondere) p-Carboxybenzyl, o-, m- oder (insbesondere) p-Methoxycarbonylbenzyl, o-, m- oder (insbesondere) p-Ethoxycarbonylbenzyl, o-, m- oder (insbesondere) p-Nitrobenzyl, o-, m- oder (insbesondere) p-Aminobenzyl, o-, m- oder (insbesondere) p-Cyanbenzyl; Cycloalkyl-alkyl wie Cyclopropyl-methyl, Cyclobutyl-methyl, Cyclopentyl-methyl, Cyclohexyl-methyl, 1- oder 2-Cyclohexyl-ethyl, Cycloheptyl-methyl, Cyclooctyl-methyl; Het-alkyl wie (insbesondere) 2- oder 3-Thienyl-methyl, 1- oder 2-(2-Thienyl)-ethyl; Ar'-alkyl wie 4-Biphenylyl-methyl, 2'-Carboxy-4-biphenylyl-methyl, 2'-Methoxycarbonyl-4-biphenylyl-methyl, 2'-Ethoxycarbonyl-4-biphenylyl-methyl, 2'-Cyan-4-biphenylyl-methyl, 2'-(1H-5-Tetrazolyl)-4-biphenylyl-methyl; R⁶-CO-alkyl wie 2-Oxo-propyl, 2-Oxo-butyl, 3-Methyl-2-oxo-butyl, 3,3-Di-methyl-2-oxo-butyl; Ar-CO-alkyl wie Benzoyl-methyl, o-, m- oder p-Carboxybenzoyl-methyl, o-, m- oder p-Methoxycarbonylbenzoyl-methyl, o-, m- oder p-Ethoxycarbonylbenzoyl-methyl, o-, m- oder p-Cyanbenzoyl-methyl, o-, m- oder p-Nitrobenzoyl-methyl, o-, m- oder p-Aminobenzoyl-methyl; Het-CO-alkyl wie 2-Thienylcarbonyl-methyl. Falls ein H-Atom im "alkyl"-Teil des Restes R³ durch COOH oder COOA ersetzt ist, steht dieser Rest bevorzugt z.B. für α-Ethoxycarbonyl-benzyl, α-Cyclohexyl-α-ethoxycarbonyl-methyl, 1-Ethoxycarbonyl-2-phenyl-ethyl.

Der Rest R⁴ ist vorzugsweise H.

Die Reste R⁵ und R⁶ sind jeweils vorzugsweise Alkyl wie Methyl oder Ethyl sowie Trifluormethyl, ferner bevorzugt Fluormethyl, Difluormethyl, Pentafluorethyl oder Heptafluorpropyl.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen und worin die nicht näher bezeichneten Reste die bei Formel I angegebenen Bedeutungen haben, worin jedoch
in Ia
- R¹: Alkyl mit 1-6 C-Atomen bedeutet;
in Ib
- R²: COOH, COOCH₃, COOC₂H₅, CN, CONH₂, NO₂ oder 1H-5-Tetrazolyl bedeutet;
in Ic
- R²: COOH, COOCH₃, COOC₂H₅, CN, CONH₂, NO₂ oder 1H-5-Tetrazolyl bedeutet und in p-Stellung steht;
in Id
- R¹: Alkyl mit 1-6 C-Atomen und
- R²: COOH, COOCH₃, COOC₂H₅, CN, CONH₂, NO₂ oder 1H-5-Tetrazolyl bedeuten.

Weiterhin sind bevorzugt Verbindungen der Formeln:
Ie sowie Iae, Ibe, Ice und Ide, die den Formeln I sowie Ia, Ib, Ic und Id entsprechen, worin jedoch zusätzlich
R³ H bedeutet;
If sowie Iaf, Ibf, Icf und Idf, die den Formeln I sowie Ia, Ib, Ic und Id entsprechen, worin jedoch zusätzlich
R³ Ar-alkyl bedeutet;
Ig sowie Iag, Ibg, Icg und Idg, die den Formeln I sowie Ia, Ib, Ic und Id entsprechen, worin jedoch zusätzlich
R³ Benzyl, Carboxybenzyl, Methoxycarbonylbenzyl, Cyanbenzyl, Nitrobenzyl oder Aminobenzyl bedeutet;
Ih sowie Iah, Ibh, Ich und Idh, die den Formeln I sowie Ia, Ib, Ic und Id entsprechen, worin jedoch zusätzlich
R³ Cycloalkyl-alkyl mit 3-8 C-Atomen in der Cycloalkylgruppe bedeutet;
Ii sowie Iai, Ibi, Ici und Idi, die den Formeln I sowie Ia, Ib, Ic und Id entsprechen, worin jedoch zusätzlich
R³ Het-alkyl bedeutet;
Ij sowie Iaj, Ibj, Icj und Idj, die den Formeln I sowie Ia, Ib, Ic und Id entsprechen, worin jedoch zusätzlich
R³ Ar'-alkyl bedeutet;
Ik sowie Iak, Ibk, Ick und Idk, die den Formeln I sowie Ia, Ib, Ic und Id entsprechen, worin jedoch zusätzlich
R³ R⁶-CO-alkyl bedeutet;
Il sowie Ial, Ibl, Icl und Idl, die den Formeln I sowie Ia, Ib, Ic und Id entsprechen, worin jedoch zusätzlich
R³ Ar-CO-alkyl bedeutet;
Im sowie Iam, Ibm, Icm und Idm, die den Formeln I sowie Ia, Ib, Ic und Id entsprechen, worin jedoch zusätzlich
R³ Het-CO-alkyl bedeutet;
In sowie Ian, Ibn, Icn und Idn, die den Formeln I sowie Ia, Ib, Ic und Id entsprechen, worin jedoch zusätzlich
R³ H, Benzyl, Carboxybenzyl, Methoxycarbonylbenzyl, Cyanbenzyl, Nitrobenzyl, Aminobenzyl, α-Carboxy-α-cyclohexyl-methyl, α-Cyclohexyl-α-methoxycarbonyl-methyl, Thienylmethyl, Carboxy-4-biphenylyl-methyl, Methoxycarbonyl-4-biphenylyl-methyl, (1H-5-Tetrazolyl)-4-biphenylyl-methyl oder 3,3-Dimethyl-2-oxo-butyl bedeutet;
Io sowie Iao, Ibo, Ico und Ido, die den Formeln I sowie Ia, Ib, Ic und Id entsprechen, worin jedoch zusätzlich
R³ H, Benzyl, p-Carboxybenzyl, α-Carboxy-benzyl, p-Methoxycarbonylbenzyl, α-Methoxycarbonylbenzyl, p-Cyanbenzyl, p-Nitrobenzyl, p-Aminobenzyl, α-Carboxy-α-cyclohexylmethyl, α-Cyclohexyl-α-methoxycarbenyl-methyl, 2-Thienylmethyl, 2'-Carboxy-4-biphenylyl-methyl, 2'-Methoxycarbonyl-4-biphenylyl-methyl, 2'-(1H-5-Tetrazolyl)-4-biphenylyl-methyl oder 3,3-Dimethyl-2-oxo-butyl bedeutet.

Insbesondere sind bevorzugt Verbindungen aller vorstehend genannten Formeln, in denen zusätzlich Y = O und/oder R⁴ = H bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-0 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃ONa oder K-tert.-Butylat in einem Alkohol wie CH₃OH oder in einem Amid wie Dimethylformamid (DMF) oder mit einem Alkalimetallhydrid wie NaH oder einem Alkalimetallalkoholat in DMF, und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallcarbonate wie Na₂CO₃ oder K₂CO₃ oder Alkalimetall-hydrogencarbonate wie NaHCO₃ oder KHCO₃.

Die Ausgangsstoffe, insbesondere diejenigen der Formel II, sind teilweise bekannt. Falls sie nicht bekannt sind, können sie nach bekannten Methoden in Analogie zu bekannten Stoffen hergestellt werden. Verbindungen der Formel III (R³ = H) sind z. B. erhältlich durch Kondensation von 3,4-Diamino-6-R⁴-1,2-dihydro-2-oxo- (bzw. -2-thioxo-) -pyridinen oder von 3,4-Diamino-2-chlor-6-R⁴-pyridinen mit Carbonsäuren der Formel R¹-COOH in Gegenwart von Polyphosphorsäure

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1-Stellung funktionell abgewandelte (durch eine Schutzgruppe geschützte) 5-Tetrazolylgruppe enthält. Als Schutzgruppen eignen sich beispielsweise: Triphenylmethyl, abspaltbar mit HCl oder Ameisensäure in einem inerten Lösungsmittel oder Lösungsmittelgemisch, z.B. Methanol oder Ether/Dichlormethan/Methanol; 2-Cyanethyl, abspaltbar mit NaOH in Wasser/THF; p-Nitrobenzyl, abspaltbar mit H₂/Raney-Nickel in Ethanol (vgl. EP-A2-0 291 969).

Es ist ferner möglich, eine Verbindung der Formel I in eine andere Verbindung der Formel I umzuwandeln, indem man einen oder mehrere der Reste R¹, R², R³, R⁴ und/oder Y in andere Reste R¹, R², R³, R⁴ und/oder Y umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Kupfer(I)cyanid) durch CN-Gruppen ersetzt und/oder Nitrilgruppen zu COOH-Gruppen oder zu CONH₂-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder freie Hydroxy- und/oder NH-Gruppen mit einem unsubstituierten oder substituierten Alkyl- oder Ar-alkylhalogenid oder mit Aldehyden wie Formaldehyd in Gegenwart eines Reduktionsmittels wie NaBH₄ oder Ameisensäure alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine NHCOR⁵- oder eine COOA-Gruppe enthält, in die entsprechende Verbindung der Formel I umgewandelt werden, die stattdessen eine NH²- oder eine COOH-Gruppe enthält. Ester-gruppen können z.B. mit NaOH oder KOH in Wasser, Wasser-THF oder Wasser-Dioxan bei Temperaturen zwischen 0 und 100° verseift werden.

Umsetzung von Nitrilen der Formel I (R² = CN oder R³ = Cyan-alkyl) mit Derivaten der Stickstoffwasserstoffsäure führt zu Tetrazolen der Formel I (R² = 1H-5-Tetrazolyl und/oder R³ = 1H-5-Tetrazolyl-alkyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgas oder Treibgasgemisch (z.B. Kohlenwasserstoffen wie Propan oder Butan oder Fluorkohlenwasserstoffen wie Heptafluorpropan) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit Die tägliche Dosierung liegt vorzugsweise zwischen etwa 0,1 und 100 mg/kg, insbesondere 1 und 50 mg/kg Körpergewicht Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.
Rf = Rf an Kieselgel (dünnschichtchromatographisch; Laufmittel: Ethylacetat/Methanol 9:1). DOI = -4,5-dihydro-4-oxo-3H-imidazo[4,5-c]pyridin.

### Beispiel 1

Man löst 19,1 g 2-Butyl-4,5-dihydro-4-oxo-1(oder 3)H-imidazo[4,5-c]pyridin (F. 285-290°; erhältlich durch Erhitzen von 3,4-Diamino-2-chlorpyridin und Valeriansäure in Polyphosphorsäure auf 100-140°, dann 170-180°) in 500 ml DMF, versetzt mit 16,6 g K₂CO₃, rührt 45 Min., tropft eine Lösung von 27,45 g p-Brommethyl-benzoesäuremethylester hinzu, rührt 16 Std. bei 20° und versetzt mit Wasser. Der ausgefallene Niederschlag wird abfiltriert, mit Wasser gewaschen, getrocknet und an Kieselgel chromatographiert. Mit Ethylacetat, dann Ethylacetat/Methanol erhält man zunächst 2-Butyl-3,5-bis-p-methoxycarbonylbenzyl-DOI (F. 124°), dann 2-Butyl-3-p-methoxycarbonylbenzyl-DOI (F. 219°).

Analog erhält man mit p-Brommethyl-benzonitril das 2-Butyl-3,5-bis-p-cyanbenzyl-DOI (F. 122,5°) und das 2-Butyl-3-p-cyanbenzyl-DOI (F. 201°).

Analog erhält man aus 4,5-Dihydro-4-oxo-2-propyl-1(oder 3)H-imidazo[4,5-c]pyridin (F. 258°; erhältlich aus 3,4-Diamino-2-chlorpyridin und Buttersäure in Polyphosphorsäure) das 3,5-Bis-p-methoxycarbonylbenzyl-2-propyl-DOI (ölig; Rf 0,51 in Ethylacetat) und das 3-p-Methoxycarbonylbenzyl-2-propyl-DOI, F. 235°.

### Beispiel 2

Analog Beispiel 1 erhält man aus 2-Butyl-5-(α-cyclohexyl-α-methoxycarbonyl-methyl)-DOI [erhältlich durch Benzylierung von 2-Butyl-4,5-dihydro-4-oxo-1(oder 3)H-imidazo[4,5-c]pyridin zur 3-Benzyl-3H-verbindung, Reaktion mit α-Brom-α-cyclohexylessigsäure-methylester zu 2-Butyl-3-benzyl-5-(α-cyclohexyl-α-methoxycarbonylmethyl)-DOI und hydrogenolytische Abspaltung der Benzylgruppe] und p-Brommethyl-benzoesäure-methylester das 2-Butyl-5-(α-cyclohexyl-α-methoxycarbonylmethyl)-3-p-methoxycarbonylbenzyl-DOI, Rf 0,63.

### Beispiel 3

Zu einer Lösung von 3,39 g 2-Butyl-3-methoxycarbonylbenzyl-DOI (F. 218-219°) in 85 ml DMF gibt man unter N₂ 1,34 g K-tert.-Butylat, rührt 10 Min. bei 20°, gibt eine Lösung von 2,16 g p-Nitrobenzylbromid in 35 ml DMF hinzu und rührt 2,5 Std. bei 20°. Man arbeitet wie üblich auf (Chromatographie an Kieselgel, Ethylacetat) und erhält 2-Butyl-3-p-methoxycarbonylbenzyl-5-p-nitrobenzyl-DOI, F. 142°.

Analog erhält man mit 2-Thienylmethylchlorid das 2-Butyl-3-p-methoxycarbonylbenzyl-5-(2-thienylmethyl)-DOI.

Analog erhält man mit α-Brom-α-cyclohexylessigsäuremethylester das 2-Butyl-5-(α-cyclohexyl-α-methoxycarbonylmehyl)-3-p-methoxycarbonylbenzyl-DOI, Rf 0,63.

Analog erhält man mit α-Brom-α-phenylessigsäuremethylester das 2-Butyl-3-p-methoxycarbonylbenzyl-5-α-methoxycarbonylbenzyl-DOI, Rf 0,47 (Ethylacetat/Hexan 9:1).

Analog erhält man mit 2-Brom-3-phenylpropionsäuremethylester das 2-Butyl-3-p-methoxycarbonylbenzyl-5-(1-methoxycarbonal-2-phenyl-ethyl)-DOI, Rf 0,64.

Analog erhält man aus 3-Methoxycarbonylbenzyl-2-propyl-DOI die nachstehenden 3-p-Methoxycabonylbenzyl-2-propyl-DOI:
5-Benzyl-
5-p-Nitrobenzyl-
5-(3,3-Dimethyl-2-oxo-butyl)-.

Analog erhält man aus 2-Butyl-3-p-cyanbenzyl-DOI mit 4'-Brommethyl-biphenyl-2-carbonsäuremethylester das 2-Butyl-3-p-cyanbenzyl-5-(2'-methoxycarbonyl-biphenyl-4-methyl)-DOI, F. 65°.

Analog erhält man aus 2-Butyl-3-p-cyanbenzyl-DOI mit Chloracetonitril das 2-Butyl-3-p-cyanbenzyl-5-cyanmethyl-DOI, F. 197°.

### Beispiel 4

Man löst 3 g 2-Butyl-4,5-dihydro-4-oxo-1(oder 3)H-imidazo[4,5-c]pyridin in 75 ml Methanol und tropft unter Rühren bei 20° eine Lösung von 0,4 g Na in 10 ml Methanol hinzu. Man rührt noch 45 Min., dampft ein, löst den Rückstand in 30 ml DMF, kühlt auf 0°, gibt bei dieser Temperatur eine Lösung von 3,7 g p-Nitrobenzylbromid hinzu und rührt 16 Std. bei 20°. Man dampft ein, arbeitet wie üblich auf und erhält nach Chromatographie (Kieselgel; Ethylacetat/Toluol 7:3) zunächst 2-Butyl-3,5-bis-p-nitrobenzyl-DOI (F. 142-143°), dann 2-Butyl-3-p-nitrobenzyl-DOI (F. 193-194°).

### Beispiel 5

Man löst 1 g 2-Butyl-3-p-methoxycarbonylbenzyl-5-p-nitrobenzyl-DOI in 50 ml Methanol , hydriert bei 20° und 1 bar an 0,5 g Pd-C (5 %) bis zum Ende der H₂-Aufnahme, filtriert und erhält nach Eindampfen und Chromatographie an Kieselgel (Ethylacetat/Methanol 9:1) 5-p-Aminobenzyl-2-butyl-3-p-methoxycarbonylbenzyl-DOI. F. 59-60°.

### Beispiel 6

Man rührt ein Gemisch von 1 g 2-Butyl-3-p-methoxycarbonylbenzyl-5-p-nitrobenzyl-DOI, 20 ml 1 N Natronlauge, 6 ml Methanol und 18 ml THF 16 Std. bei 20°, säuert mit Salzsäure an, arbeitet wie üblich auf und erhält 2-Butyl-3-p-carboxybenzyl-5-p-nitrobenzyl-DOI, F. 170°.

Analog erhält man durch Verseifung der entsprechenden Methylester die nachstehenden DOI:
5-p-Aminobenzyl-2-butyl-3-p-carboxybenzy-, F. 130°
2-Butyl-3-p-carboxybenzyl-, F. 249°
2-Butyl-3,5-bis-p-carboxybenzyl-, F. 150°
3-p-Carboxybenzyl-2-propy-, F. 289°
3,5-Bis-p-carboxybenzyl-2-propyl-, F. 209°
5-Benzyl-3-p-carboxybenzyl-2-butyl-, F. 212°; K-Salz, F. > 300°
3-p-Carboxybenzyl-5-p-nitrobenzyl-2-propyl-, F. 300°
2-Butyl-3-p-carboxybenzyl-5-(2-thienylmethyl)-, F. 201°
3-p-Carboxybenzyl-5-(3,3-dimethyl-2-oxo-butyl)-2-propyl-, F. 195°
2-Butyl-3-p-carboxybenzyl-5-α-carboxy-α-cyclohexy-methyl-, F. 195°
2-Butyl-3-p-carboxybenzyl-5-α-carboxybenzyl-, Sesquihydrat, F. 234°
2-Butyl-3-p-carboxybenzyl-5-(1-carboxy-2-phenyl-ethyl-)-, F. 253°.

### Beispiel 7

Setzt man 2-Butyl-3-p-cyanbenzyl-5-(2'-methoxycarbonylbiphenylyl-4-methyl)-DOI analog Beispiel 6 mit Natronlauge/Methanol/THF um, so erhält man als Hauptprodukt 2-Butyl-3-p-carbamoylbenzyl-5-(2'-carboxy-biphenylyl-4-methyl)-DOI, F 241°.

### Beispiel 8

a) Ein Gemisch von 4,21 g 2-Butyl-3,5-bis-p-cyanbenzyl-DOI, 41,2 g Trimethylzinnazid und 300 ml Toluol wird 72 Std. gekocht und eingedampft. Man rührt den Rückstand mit 100 ml methanolischer Salzsäure 2 Std. bei 20°, arbeitet wie üblich auf (gesättigte NaCl-Lösung/Dichlormethan) und erhält 2-Butyl-3,5-bis-[p-(1H-5-tetrazolyl)-benzyl]-DOI, F. 272°.
   Analog erhält man aus 2-Butyl-3-p-cyanbenzyl-DOI das 2-Butyl-3-[p-(1H-5-tetrazolyl-benzyl]-DOI.
   Analog erhält man aus 2-Butyl-3-p-cyanbenzyl-5-(2'-methoxycarbonyl-biphenylyl-4-methyl)-DOI das 2-Butyl-5-(2'-methoxycarbonyl-biphenylyl-4-methyl)-3-[p-(1H-5-tetrazolyl)-benzyl]-DOI, F. 154°.
   Analog erhält man aus 2-Butyl-3-p-cyanbenzyl-5-cyanmethyl-DOI das 2-Butyl-3-[p-(1H-5-tetrazolyl)-benzyl]-5-(1H-tetrazolyl-methyl)-DOI, F. 276° (Zersetzung).

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

1. Imidazopyridinderivate der Formel I 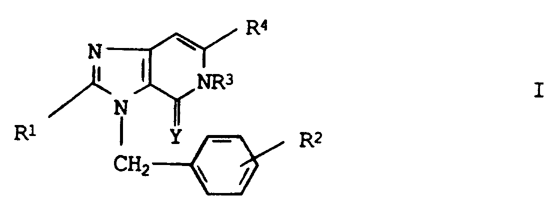 worin
R¹ H oder A,
R² H, Hal, OH, OA, COOH, COOA, CONH₂, CN, NO₂, NH₂, NHA, N(A)₂, NHCOR⁵, NHSO₂R⁵ oder 1H-5-Tetrazolyl,
R³ H, Cyan-alkyl, Ar-alkyl, Cycloalkyl-alkyl mit 3-8 C-Atomen in der Cycloalkylgruppe, Het-alkyl, Ar'-alkyl, R⁶-CO-alkyl, Ar-CO-alkyl oder Het-CO-alkyl mit jeweils 1-6 C-Atomen im "alkyl"-Teil, wobei ein H-Atom im "alkyl"-Teil durch eine COOH- oder eine COOA-Gruppe substituiert sein kann,
R⁴ H oder Hal,
R⁵ und R⁶ jeweils Alkyl mit 1-6 C-Atomen, worin auch ein oder mehrere H-Atom(e) durch F ersetzt sein kann (können),
Y O oder S,
A Alkyl, Alkenyl oder Alkinyl mit jeweils bis zu 6 C-Atomen,
Ar eine unsubstituierte oder eine durch Hal, R⁵, OH, OA, COOH, COOA, CN, NO₂, NH₂, NHA, N(A)₂, NHCOR⁵, NHSO₂R⁵ oder 1H-5-Tetrazolyl mono-substituierte Phenylgruppe,
Ar' eine durch Ar substituierte Phenylgruppe,
Het einen fünf- oder sechsgliedrigen heteroaromatischen Rest mit 1 bis 3 N-, O- und/oder S-Atomen, der auch mit einem Benzol- oder Pyridinring kondensiert sein kann und
Hal F, Cl, Br oder I bedeuten,
sowie ihre Salze.

2. a)2-Butyl-5-benzyl-3-p-carboxybenzyl-4,5-dihydro-4-oxo-3H-imidazo[4,5-c]pyridin und dessen Salze;
b)2-Butyl-3-p-carboxybenzyl-5-(2-thienylmethyl)-4,5-dihydro-4-oxo-3H-imidazo[4,5-c]pyridin und dessen Salze;
c)5-p-Aminobenzyl-2-butyl-3-p-carboxybenzyl-4,5-dihydro-4-oxo-3H-imidazo[4,5-c]pyridin und dessen Salze.

3. Verfahren zur Herstellung von Imidazopyridinen der Formel I nach Anspruch 1 sowie ihrer Salze, dadurch gekennzeichnet, daß man eine Verbindung der Formel II 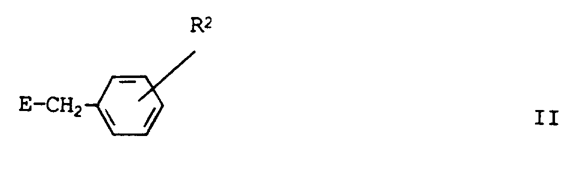 worin
E Cl, Br, I oder eine freie oder reaktionsfähig funktionell abgewandelte OH-Gruppe bedeutet und
R² die in Anspruch 1 angegebene Bedeutung hat,
mit einer Verbindung der Formel III 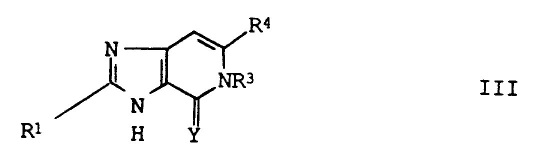 worin
R¹, R³, R⁴ und Y die in Anspruch 1 angegebenen Bedeutungen haben,
umsetzt
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹, R², R³, R⁴ und/oder Y in einen oder mehrere andere Reste R¹, R², R³, R⁴ und/oder Y umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindung der Formel I nach Anspruch 1 und ihre physiologisch unbedenklichen Säureadditionssalze zur Bekämpfung von Krankheiten.

7. Verwendung von Verbindungen der Formel I nach Anspruch 1 und/oder ihrer physiologisch unbedenklichen Säureadditionssalze zur Herstellung eines Arzneimittels.

8. Verwendung von Verbindungen der Formel I nach Anspruch 1 und/oder ihrer physiologisch unbedenklichen Säureadditionssalze bei der Bekämpfung von Krankheiten.
